# EUROPEAN PATENT APPLICATION

(11) **EP 1 885 070 A1**
(43) Date of publication of application: **06.02.2008**
(21) Application number: 07113322.7
(22) Date of filing: 27.07.2007
(51) Int. Cl.: H04B 7/06

(54) **Half-duplex communication system**

(30) Priority: 01.08.2006 GB 0615261
(71) Applicant: Zarlink Semiconductor Limited, Swindon, Wiltshire SN2 2QW (GB)
(72) Inventor: Coombs, Timothy John, Cardiff, Wales CF10 6UQ (GB)
(74) Representative: Robinson, John Stuart

(57) **Abstract**

First and second transceivers (1, 2) are provided for half-duplex communication via a plurality of communication channels, for example by means of antenna diversity (7, 8). The first transceiver (1) comprises a receiver (4) and transmitter (3) together with a switching arrangement (6) for selecting between two or more channels, for example in the form of antennae (7, 8) disposed at different locations, providing different polarisations or having different beam patterns. A controller (9) controls the switch (6) so that the aerial selection is changed after each receive and transmit period. If the receiver (4) indicates unacceptable reception, for example resulting from the absence of at least part of a packet or the corruption of data within the packet, the transmitter (3) retransmits the packet which it sent in the preceding transmission period via the previously selected antenna.

## Description

The present invention relates to a half-duplex communication system.

It is known to make use of antenna diversity in communication systems for improving the quality or range of communication. In a spatial antenna diversity system, two or more antennae are disposed at different locations. In a polarisation antenna diversity system, two or more antennae providing different polarisations of transmission and reception are provided. In a beam antenna diversity system, two or more antennae having different beam directions and/or patterns are used.

In such systems, the intention is to make use of the antenna which is currently providing the greatest signal strength (for reception or transmission or both). One way of achieving this is to measure the signal strength available from each antenna and to select for use the antenna which provides the greatest signal strength. Another technique is to perform a measuring sequence from time to time in which the different antennae are selected in turn and the resulting signal strength measured so that the antenna providing the greatest signal strength can be selected and used until the next selection process. Switching may be performed between a plurality of antennae and the input of a single receiver. Alternatively, each antenna may be connected to a respective receiver and switching may be performed at the outputs of the receivers. The single receiver arrangement is cheaper in terms of hardware but the multiple receiver arrangement allows the signal strengths from the antennae to be determined without interrupting the received signal.

In a half-duplex communication system, two (or more) transceivers are interconnected by a communication channel which permits the transmission of signals in one direction at a time. In the case of two users, communication is possible in either direction but not in both directions simultaneously. In such a half-duplex system, the same antenna may be used both for receiving and transmitting because the conditions should be "reciprocal".

Half-duplex communication systems have many applications, one example of which is in providing communication between a medical implant device and an external device.

Examples of known transceiver devices, which may be used in medical implant devices but which may also be used in other applications, are ZL70100 and ZL70101 available from Zarlink UK. These devices include a media access controller (MAC) for managing various aspects of communication, such as error detection and correction and flow control. Two such devices may be used to engage in a communication session and perform a "ping-pong" transmission sequence, such that one of the devices transmits a data packet and then activates its receiver to await reception of a packet from the other device. If no packet is received after a predetermined time or if a corrupted or incomplete packet is received, the first device retransmits the original packet with an indication that a response or a correct response was not received. When a packet is correctly received, this is indicated in the next transmission so that the other device may then send a new packet.

Such devices cannot conveniently be used with traditional diversity switching control methods. In particular, only two such devices may be involved in a communication session at any one time. Thus, if diversity switching were to be performed at receiver outputs, changing from one receiver to another would necessitate stopping the existing session and starting a new session, which is inefficient and prevents reaction to rapid changes in conditions. Conversely, if antenna switching were used with a single receiver, monitoring of a currently non-selected antenna would require switching away from the antenna currently in use, with the danger of disturbing packets which are in the middle of being transmitted or received. This would result in a reduction in data rate even in good communication conditions. Also, additional software would be required to read the signal strength from the different antennae and to make a decision to perform switching.

According to first aspect of the invention, there is provided a half-duplex communication system comprising a first transceiver for performing half-duplex communication with a remote second transceiver via a plurality of communication channels, the first transceiver comprising a first receiver, a first transmitter, a switching arrangement arranged to select any one of the channels at a time, and a controller, the second transceiver comprising a second receiver and a second transmitter, characterised in that the controller is arranged to: control the first receiver and the first transmitter to perform a series of communication cycles, each of which comprises at least one first reception period and at least one first transmission period; control the switching arrangement to change the selected channel in response to completion of each communication cycle; and cause the first transmitter to retransmit, in response to an unacceptable reception event, at least part of a message, which was previously transmitted by the first transmitter via a channel different from the currently selected channel, the second receiver being arranged, during second reception periods, to receive via all of the channels simultaneously and the second transmitter being arranged, during second transmission periods which alternate with the second reception periods, to transmit via all of the channels simultaneously.

Each communication cycle may comprise a single first reception period and a single first transmission period. Each communication cycle may comprise a single first reception period followed by a single first transmission period.

The unacceptable reception event may comprise at least one of: failure by the first or second receiver to receive an expected message; receipt by the first or second receiver of an incomplete and/or corrupted message; and receipt by the first receiver of a request for retransmission. The controller may be further arranged, in response to the unacceptable reception event, to control the switching arrangement to change the selected channel.

According to a second aspect of the invention, there is provided a half-duplex communication system comprising a first transceiver for performing half-duplex communication with a remote second transceiver via a plurality of communication channels, the first transceiver comprising a first receiver, a first transmitter, a switching arrangement arranged to select any one of the channels at a time, and a controller, the second transceiver comprising a second receiver and a second transmitter, characterised in that the controller is arranged to: control the first receiver and the first transmitter to perform alternating first reception and transmission periods; control the switching arrangement to change the selected channel in response to an unacceptable reception event; and cause the first transmitter to retransmit, in response to the unacceptable reception event, at least part of a message, which was previously transmitted by the first transmitter via a channel different from the currently selected channel, the second receiver being arranged, during second reception periods, to receive via all of the channels simultaneously and the second transmitter being arranged, during second transmission periods which alternate with the second reception periods, to transmit via all of the channels simultaneously, the unacceptable reception event comprising at least one of: failure by the first or second receiver to receive an expected message; receipt by the first or second receiver of an incomplete or corrupted message; and receipt by the first receiver of a request for retransmission.

The second transmitter may be arranged to retransmit at least part of a further message, which was previously transmitted by the second transmitter, in response to at least one of: failure by the first or second receiver to receive a further expected message; receipt by the first or second receiver of a further incomplete or corrupted message; and receipt by the second receiver of a request for retransmission.

The first transmitter may be arranged to retransmit at least part of the message which was transmitted in the preceding first transmission period.

The channels may be wireless channels. The switching arrangement may be arranged to select any one of a plurality of antennae at a time. The antennae may have different ones or combinations of different locations, different frequencies, different polarisations and different beam patterns. The second receiver and the second transmitter may be connected to a common antenna arrangement.

The switching arrangement and the controller may be arranged to select the channels in turn in a repeating cycle.

The first and second receivers and the first and second transmitters may be arranged to receive and transmit data packets. The first transmitter may be arranged, when retransmitting, to retransmit a complete data packet.

The second transceiver may comprise part of a body implant.

Such an arrangement provides relatively simple diversity operation without requiring any substantial additional hardware or software for its implementation. During good propagation conditions, such an arrangement is capable of operating at the maximum communication rate. For worsening conditions, such as increased propagation distance, communication remains possible up to the maximum distance supported by the channel having the best propagation characteristics at the time.

The invention will be further described, by way of example, with reference to the accompanying drawings, in which:
Figure 1 is a block schematic diagram of a half-duplex communication system constituting an embodiment of the invention; and
Figure 2 is a graph of normalised data rate against normalised range illustrating the performance of the embodiment of Figure 1.

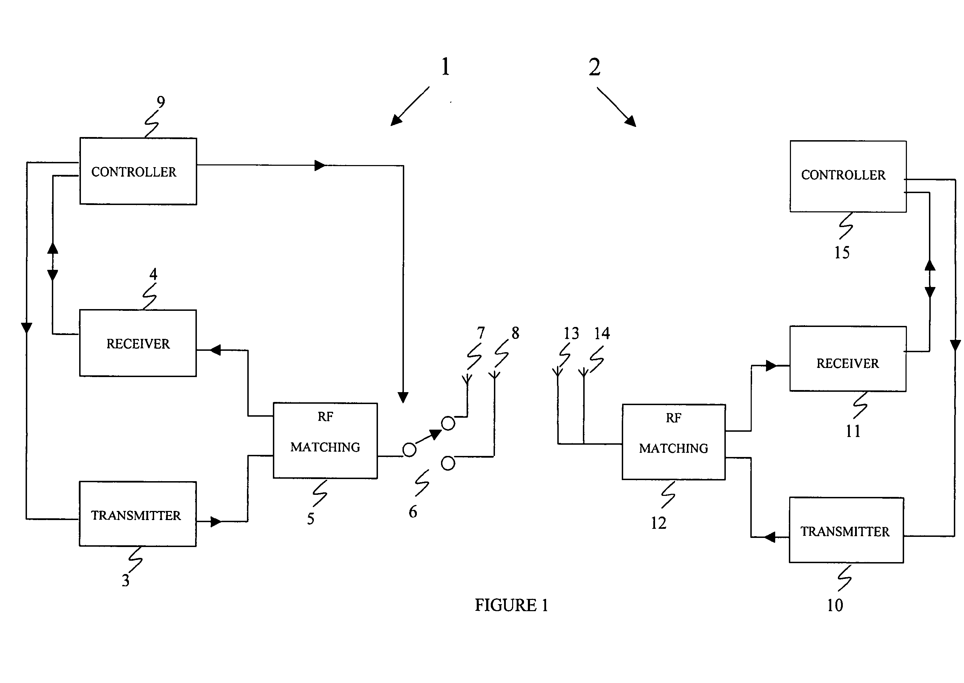

The half-duplex communication system shown in Figure 1 comprises a first transceiver 1 and a second transceiver 2. Such a system may be used for any suitable purpose and the example illustrated in Figure 1 is intended for half-duplex communication between an external arrangement including the transceiver 1 and a body implant including the transceiver 2. The system performs half-duplex wireless communication using automatic handshaking and antenna diversity. Each of the transceivers 1 and 2 may be partially embodied by medical implant communication service (MRCS) transceiver devices, for example of the ZL70100 or ZL70101 type mentioned hereinbefore.

The first transceiver 1 comprises a first transmitter 3 and a first receiver 4 connected via a radio frequency (RF) matching arrangement 5 to an electronic switching arrangement 6. The switching arrangement 6 is arranged to select from among a plurality of antennae so as to perform antenna diversity. In the embodiment illustrated in Figure 1, for the sake of simplicity, only two such antennae 7 and 8 are shown although more such antennae may be provided as appropriate. The switching arrangement 6 is arranged to select one of the antennae at a time. The antennae provide diversity by having different locations and/or different polarisations and/or different beam patterns and/or directions.

The transceiver 1 further comprises a controller 9 which controls the operation of the transmitter 3, the receiver 4, and the switching arrangement 6. The controller 9 enables the transmitter 3 and the receiver 4 to perform interlaced or alternating transmission and reception periods and causes the switching arrangement 6 to change the antenna selection in accordance with the desired mode of operation.

The second transceiver 2 comprises a second transmitter 10 and a second receiver 11 connected via an RF matching arrangement 12 to a common antenna arrangement. One or more antennae may be provided and the transceiver 2 shown in Figure 1 has two such antennae 13 and 14. Another controller 15 controls the transmitter 10 and the receiver 11 to perform alternate or interlaced transmission and reception periods with all of the antennae 13, 14 being used during each such period.

When the system shown in Figure 1 is used for communication with a body implant, the transceiver 2 forms part of the body implant and is connected to other devices as appropriate. For example, body function sensors may be provided in the implant and may supply data to the transmitter 10 for transmission to the transceiver 1 for monitoring. The transceiver 1 may form part of a monitoring station and may be used, for example, to transmit instructions to the implant to control its operation. Alternatively or additionally, the implant may include one or more actuators whose operation is at least partially controlled by data sent by the transceiver 1.

The half-duplex communication system performs automatic handshaking with antenna diversity. Each of the controllers 9 and 15 controls the associated receiver and transmitter to perform a series of communication cycles such that each of the communication cycles comprises at least one reception period followed by at least one transmission period. For example each such communication cycle may comprise a single reception period followed by a single transmission period.

During a reception period of the receiver 4 controlled by the controller 9, the transceiver 1 expects to receive a message from the transceiver 2, for example comprising one or more packets of data. If the receiver 4 receives a complete and uncorrupted message from the transceiver 2, the controller 9 causes the transmitter 3 to send a return message which may or may not provide acknowledge of correct reception and which may include one or more data packets for the transceiver 2.

The controller 15 synchronises the transceiver 2 such that each reception period of the receiver 11 contains a corresponding transmission period of the transmitter 3. if the receiver 11 receives a complete and uncorrupted message from the transceiver 1, the controller 15 causes the transmitter 10 to transmit a reply during the next transmission period. Again, the reply may contain an acknowledgement of correct receipt of the message from the transceiver 1 and may comprise one or more data packets for transmission to the receiver 4.

If either of the receivers 5 and 11 receives, during any of its reception periods, an incomplete or corrupted message or fails to receive any message during the expected reception period, the receiver signals the corresponding controller 9 or 15 that an "unacceptable reception event" has occurred. The controller then causes the corresponding transmitter to send a message to the other transceiver signalling unacceptable reception and typically containing a request for retransmission of the message whose reception was unacceptable. The controller may cause the transmitter to request a complete retransmission of the previous message from the other transceiver or may request only partial retransmission of any part of the message which was not acceptably or correctly received. When the remote receiver receives such a message, it retransmits part or all of the message which it previously transmitted and which was not correctly received.

In order to provide antenna diversity, the transceiver 1 may operate in either or both of two modes of operation. According to the first mode of operation, the controller 9 automatically causes the switching arrangement 6 to change the selected antenna after the completion of each communication cycle by the receiver 4 and the transmitter 3. In particular, changing of the selected antenna occurs entirely automatically as a result of completion of each communication cycle by the transceiver 1. The switching arrangement 6 cycles through the available antennae in a repeating cycle.

When both of the antennae 7 and 8 provide adequate communication with the transceiver 2, the receivers 4 and 11 correctly receive the messages transmitted by the transmitters 10 and 3, respectively, so that the messages are transmitted without the need for repetition. The dataflow between the transceivers 1 and 2 thus proceeds at the maximum rate and switching between the antennae 7 and 8 has no effect.

During operation of the communication system, the propagation conditions between the transceivers 1 and 2 may deteriorate. For example, received signal strength may decrease because of increasing separation between the transceivers or because of other causes, such as relative changes in orientation. As the received signal strength reduces, a point is reached where the signal strength for one of the antennae 7 and 8 becomes inadequate for correct transmission or reception. When the "inadequate" antenna is selected, the receiver 4 or 11 signals to the corresponding controller 9 or 15 an unacceptable reception event. As mentioned hereinbefore, this may result from failure to receive a message within the allotted reception period, from reception of only part of a message, or from corruption of a message such that it is not correctly decipherable. For example, when the receiver 4 suffers such an unacceptable reception event, or receives a request for retransmission from the transmitter 10, it signals the controller 9 which, in turn, causes the transmitter 3 to transmit the message, or part of the message, which was transmitted in the preceding transmission period of the transceiver 1. However, at the end of the previous communication cycle of the transceiver 1, the controller 9 caused the switching arrangement 6 to select a different antenna. Accordingly, the current communication cycle is performed via the "adequate" antenna.

In the case of a two antenna diversity arrangement as illustrated in the example of Figure 1, if one "communication channel" corresponding to one of the antennae 7 and 8 continues to be inadequate, then two-way communication continues but at substantially half the maximum data rate. For reception conditions between the two extremes, the data rate is between the maximum rate and half the maximum rate.

If reception conditions deteriorate further, there comes a point where the signal strength is inadequate for communication by any antenna and communication then ceases. The ultimate range is substantially the same as that for the antenna providing the higher signal strength.

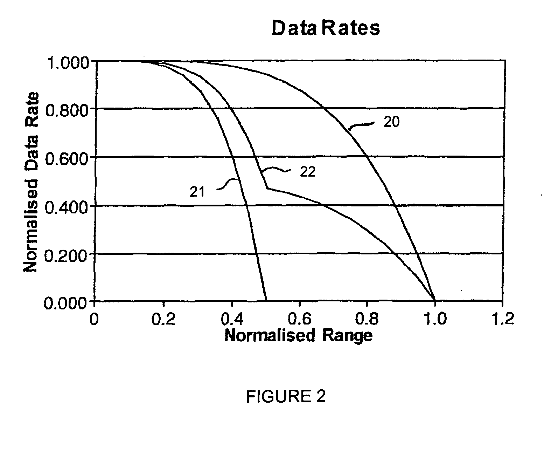

Figure 2 illustrates the data rates which may be achieved in the case where there is a 6dB signal strength difference between the antennae 5 and 6. The curve 20 illustrates the normalised data rate against normalised range for the antenna providing the higher signal strength whereas the curve 21 illustrates the data rate for the antenna providing the lower signal strength. The curve 22 illustrates the performance for the system of Figure 1. Thus, as the range increases from zero, the system initially provides communication at the maximum data rate. As the range increases further, the system provides communication at a data rate between the rates for the higher signal strength and lower signal strength antennae but with the same ultimate range as for the higher strength antenna. In situations where, for example, multi-path reception is changing or relative polarisation is changing, for example when an implanted patient is moving or rolling over, the system provides the same maximum range of operation as, for example, a more complex antenna diversity system. Although the data rate may not be as high as for a more sophisticated system, this arrangement is relatively simple and inexpensive.

In the second mode of operation, changing the selection of the antennae 7, 8 by the switching arrangement 6 is controlled by the controller 9 only when an unacceptable reception event, as described hereinbefore, happens. Thus, at the start of a communication session, the controller 9 controls the switching arrangement 6 to select arbitrarily one of the antennae 7 and 8. The selected antenna is then used for communication with the transceiver 2 unless and until the receiver 4 signals to the controller 9 that an unacceptable reception event has happened. The controller 9 then causes the switching arrangement 6 to select a different one of the antennae, in the present example the other of the antennae 7 and 8, for further communication between the transceivers. The newly selected antenna is then used for further communication unless and until another unacceptable reception event happens, in which case the antenna selection is changed.

Depending on the reception conditions, this mode of operation may allow faster data rates to be obtained from the communication system. For example, where the propagation conditions for the antennae are changing slowly, the data rate may be equal to or close to the maximum possible rate. The maximum range of operation is the same as for the first mode and the same for more complex antenna diversity systems of known type. However, in both modes of operation, antenna switching is employed so that the transceivers may be relatively simple and less expensive than known arrangements. Although data rates may not be as high as for the more sophisticated systems of known type, the maximum range may be the same and acceptable performance throughout the range may be achieved for many applications.

The system may be arranged to operate in both of the above-described modes simultaneously. For example, each communication cycle may comprise a plurality of transmission periods alternating with a plurality of reception periods with the antenna selection being changed at the end of each communication cycle. However, if an unacceptable reception event occurs during a communication cycle, the controller 9 may then change the antenna selection before the end of the communication cycle. At the end of that communication cycle, the antenna selection may be changed automatically or may remain the same for the next communication cycle.

## Claims

1. A half duplex communication system comprising a first transceiver (1) for performing half-duplex communication with a remote second transceiver (2) via a plurality of communication channels (7, 8), the first transceiver (1) comprising a first receiver (4), a first transmitter (3), a switching arrangement (6) arranged to select any one of the channels (7, 8) at a time, and a controller (9), the second transceiver (2) comprising a second receiver (11) and a second transmitter (10), **characterised in that** the controller (9) is arranged to: control the first receiver (4) and the first transmitter (3) to perform a series of communication cycles, each of which comprises at least one first reception period and at least one first transmission period; control the switching arrangement (6) to change the selected channel (7, 8) in response to completion of each communication cycle; and cause the first transmitter (3) to retransmit, in response to an unacceptable reception event, at least part of a message, which was previously transmitted by the first transmitter (3) via a channel different from the currently selected channel, the second receiver (11) being arranged, during second reception periods, to receive via all of the channels simultaneously and the second transmitter (10) being arranged, during second transmission periods which alternate with the second reception periods, to transmit via all of the channels (7, 8) simultaneously.

2. A system as claimed in claim 1, **characterised in that** each communication cycle comprises a single first reception period and a single first transmission period.

3. A system as claimed in claim 2, **characterised in that** each communication cycle comprises a single first reception period followed by a single first transmission period.

4. A system as claimed in any one of the preceding claims, **characterised in that** the unacceptable reception event comprises at least one of: failure by the first or second receiver (4, 11) to receive an expected message; receipt by the first or second receiver (4, 11) of an incomplete and/or corrupted message; and receipt by the first receiver (4) of a request for retransmission.

5. A system as claimed in claim 4, **characterised in that** the controller (9) is further arranged, in response to the unacceptable reception event, to control the switching arrangement (6) to change the selected channel.

6. A half-duplex communication system comprising a first transceiver (1) for performing half-duplex communication with a remote second transceiver (2) via a plurality of communication channels (7, 8), the first transceiver (1) comprising a first receiver (4), a first transmitter (3), a switching arrangement (6) arranged to select any one of the channels (7, 8) at a time, and a controller (9), the second transceiver (2) comprising a second receiver (11) and a second transmitter (10), **characterised in that** the controller (9) is arranged to: control the first receiver (4) and the first transmitter (3) to perform alternatively first reception and transmission periods; control the switching arrangement (6) to change the selected channel in response to an unacceptable reception event; and cause the first transmitter (3) to retransmit, in response to the unacceptable reception event, at least part of a message, which was previously transmitted by the first transmitter (3) via a channel different from the currently selected channel, the second receiver (11) being arranged, during second reception periods, to receive via all of the channels (7, 8) simultaneously and the second transmitter (10) being arranged, during second transmission periods which alternate with the second reception periods, to transmit via all of the channels (7, 8) simultaneously, the unacceptable reception event comprising at least one of: failure by the first or second receiver (4, 11) to receive an expected message; receipt by the first or second receiver (4, 11) of an incomplete or corrupted message; and receipt by the first receiver (4) of a request for retransmission.

7. A system as claimed in any one of the preceding claims, **characterised in that** the second transmitter (10) is arranged to retransmit at least part of a further message, which was previously transmitted by the second transmitter (10), in response to at least one of: failure by the first or second receiver (4, 11) to receive a further expected message; receipt by the first or second receiver (4, 11) of a further incomplete or corrupted message; and receipt by the second receiver (11) of a request for retransmission.

8. A system as claimed in any one of the preceding claims, **characterised in that** the first transmitter (3) is arranged to retransmit at least part of the message which was transmitted in the preceding first transmission period.

9. A system as claimed in any one of the preceding claims, **characterised in that** the channels are wireless channels.

10. A system as claimed in claim 9, **characterised in that** the switching arrangement (6) is arranged to select any one of a plurality of antennae (7, 8) at a time.

11. A system as claimed in claim 10, **characterised in that** the antennae (7, 8) have different ones or combinations of different locations, different frequencies, different polarisations and different beam patterns.

12. A system as claimed in any one of claims 9 to 11, **characterised in that** the second receiver (11) and the second transmitter (10) are connected to a common antenna arrangement (13, 14).

13. A system as claimed in any one of the preceding claims, **characterised in that** the switching arrangement (6) and the controller (9) are arranged to select the channels (7, 8) in turn in a repeating cycle.

14. A system as claimed in any one of the preceding claims, **characterised in that** the first and second receivers (4, 11) and the first and second transmitters (3, 10) are arranged to receive and transmit data packets.

15. A system as claimed in claim 14, **characterised in that** the first transmitter (3) is arranged, when retransmitting, to retransmit a complete data packet.

16. A system as claimed in any one of the preceding claims, **characterised in that** the second transceiver (2) comprises part of a body implant.
